# EUROPEAN PATENT SPECIFICATION

(11) **EP 4 302 718 B1**
(45) Date of publication and mention of the grant of the patent: **27.08.2025**
(21) Application number: 22315136.6
(22) Date of filing: 06.07.2022
(51) Int. Cl.: A61B 34/20, A61B 90/00, A61B 17/00

(54) **SURGICAL NAVIGATION SYSTEM**
CHIRURGISCHES NAVIGATIONSSYSTEM
SYSTÈME DE NAVIGATION CHIRURGICALE

(43) Date of publication of application: 10.01.2024
(73) Proprietor: Ecential Robotics, 38610 Gieres (FR)
(72) Inventor: Sene, Jérémy, 38610 GIÈRES (FR)
(74) Representative: INNOV-GROUP

(56) References cited:
- US-A1- 2003 209 096
- US-A1- 2015 018 672

## Description

### Technical field

The invention relates to the technical field of surgical navigation systems.

The invention is notably applicable to navigate a surgical tool during a surgical intervention.

### Background art

A surgical navigation system known from prior art, notably from US 2003/0209096 A1, comprises:
- a surgical tool, having a longitudinal axis;
- an optical tracking system, comprising:
   a light source, configured to emit infrared radiations;
   an optical tracker, arranged to track a pose of the surgical tool from a known longitudinal axis which is the longitudinal axis of the surgical tool;
   cameras, configured to detect infrared radiations, and arranged to capture images of the optical tracker;
   a data processor, configured to compute the pose of the surgical tool;
- a calibrating device, configured to calibrate the surgical tool tracked by the optical tracking system; the calibrating device comprising:
   a support, arranged to support the surgical tool so that the longitudinal axis of the surgical tool remains fixed as the surgical tool is rotated on the support about a rotating axis;
   a tool stop, operable to retain a tip of the surgical tool in known positions.

The data processor is configured to compute the pose of the surgical tool from the captured images of the optical tracker, by determining the rotating axis and the positions of the tip.

Such a surgical navigation system from the prior art is not entirely satisfactory because the application thereof is not operational for asymmetrical surgical tools. Moreover, the accuracy of the pose of the surgical tool is limited since only the rotating axis and the tip are taken into account. Thus, the general shape (e.g. profile) of the surgical tool is not considered.

Another surgical navigation system known from prior art, notably from US 2004/0039402 A1, comprises:
- an asymmetrical surgical tool (e.g. chisel), having a cutting edge;
- an optical tracking system, comprising:
   an optical tracker, arranged to track a pose of the surgical tool from a known longitudinal axis which is an axis vector of the chisel;
   cameras, arranged to capture images of the optical tracker;
   a data processor, configured to compute the pose of the surgical tool;
- a calibrating device, configured to calibrate the surgical tool tracked by the optical tracking system; the calibrating device comprising a groove arranged to receive the cutting edge of the surgical tool, the geometry of the groove being known.

The surgical tool is continuously rotated into the groove about a rotating axis which corresponds to the cutting edge.

The data processor is configured to compute the pose of the surgical tool from the captured images of the optical tracker, by determining the rotating axis and calculating the radius of the chisel grip.

The chisel grip may initially have a random radius. Then, the data processor has to complete calculations with error correction. In the event of inaccurate calibration, the accuracy of calibration can be increased by matching the chisel grip with the aid of manually inputted radius.

Such a surgical navigation system from the prior art is not entirely satisfactory because the accuracy of the pose of the surgical tool is limited. Indeed, the general shape (e.g. profile) of the surgical tool is not accurately considered.

### Disclosure of the invention

The invention aims to completely or partly overcome the aforementioned drawbacks. To this end, one subject of the invention is a surgical navigation system, comprising:
- a surgical tool, having a profile;
- an optical tracking system, comprising:
   a light source, configured to emit infrared radiations;
   an optical tracker, arranged to track a pose of the surgical tool from a known longitudinal axis;
   a camera, preferably stereoscopic, configured to detect infrared radiations, and arranged to capture images of the optical tracker;
   a data processor, configured to compute the pose of the surgical tool;
- a calibrating device, configured to calibrate the surgical tool tracked by the optical tracking system; the calibrating device comprising:
   a reflector, arranged to reflect infrared radiations emitted by the light source;
   a holder, arranged to hold the surgical tool between the light source and the reflector, the holder having a known relative position with respect to the reflector;
wherein the light source is arranged to irradiate the profile of the surgical tool held by the holder, the camera is arranged to detect the infrared radiations which are reflected by the surgical tool and by the reflector so as to capture images of the profile of the surgical tool, and the data processor is configured to compute the pose of the surgical tool from the captured images of both the optical tracker and the profile of the surgical tool.

Thus, such a surgical navigation system according to the invention makes it possible to improve the accuracy of the pose of the surgical tool, even for an asymmetrical tool, compared to prior art. Indeed, the calibrating device allows the camera to capture the profile of the surgical tool. Thus, the pose of the surgical tool is computed from the real profile of the surgical tool (captured by the camera) in the sense that the profile does not need to be extrapolated by calculation methods. The profile of the surgical tool can be observed in the captured images of the camera insofar as the reflection coefficient of the reflector (dedicated to this function) is much higher than the reflection coefficient of the surgical tool.

The surgical navigation system according to the invention may include one or more of the following features.

According to one feature of the invention:
- the surgical tool is mobile with respect to the holder in a set of defined positions which are defined to identify the symmetry or the asymmetry of the surgical tool;
- the data processor is configured to compute the pose of the surgical tool from the captured images of both the optical tracker and a set of profiles of the surgical tool, the set of profiles corresponding to the set of defined positions.

Thus, one advantage afforded is that the calibration of the surgical tool can be easily carried out by a surgeon. Indeed, the calibration of the surgical tool can be performed manually by moving the surgical tool with respect to the holder in the set of defined positions.

According to one feature of the invention:
- the holder is mobile to hold the surgical tool in a set of defined positions which are defined to identify the symmetry or the asymmetry of the surgical tool, the holder is preferably mobile in rotation about three axes defining a rectangular trihedron;
- the data processor is configured to compute the pose of the surgical tool from the captured images of both the optical tracker and a set of profiles of the surgical tool, the set of profiles corresponding to the set of defined positions.

Thus, one advantage afforded is that the calibration of the surgical tool can be performed automatically by moving the holder in the set of defined positions.

According to one feature of the invention:
- the surgical tool has a known axis of symmetry;
- the known longitudinal axis is the axis of symmetry of the surgical tool;
- the optical tracker is arranged on the surgical tool.

Thus, one advantage afforded is that the surgical navigation system is operable when the surgical tool is already equipped with a dedicated optical tracker.

According to one feature of the invention, the surgical navigation system comprises a robotic arm; wherein:
- the holder comprises a tool guide mounted on the robotic arm, the tool guide extending along a guiding axis so as to guide the surgical tool;
- the known longitudinal axis is the guiding axis of the tool guide;
- the optical tracker is arranged on the tool guide.

Thus, one advantage afforded is that the surgical navigation system is operable in the presence of a robotic arm intended to be equipped with the surgical tool.

According to one feature of the invention, the surgical navigation system comprises a universal adapter on which the surgical tool is removably mounted; wherein:
- the universal adapter has a known axis of symmetry;
- the known longitudinal axis is the axis of symmetry of the universal adapter;
- the optical tracker extends over the universal adapter.

Thus, one advantage afforded is that the surgical navigation system is operable in the presence of a universal adapter equipped with an optical tracker, without knowing any prior information about a possible axis of symmetry of the surgical tool.

According to one feature of the invention, the holder comprises a carrier designed to carry the universal adapter.

Thus, one advantage afforded is that the carrier can easily be implemented in the presence of a universal adapter.

According to one feature of the invention, the surgical navigation system comprises a robotic arm; wherein the carrier is mounted on the robotic arm.

Thus, one advantage afforded is that the calibration of the surgical tool can easily be implemented by reducing the distance between the carrier and the robotic arm, compared to a situation where the carrier would be located far away from the robotic arm. In other words, one advantage afforded is that of avoiding to move the robotic arm towards the carrier in order to reduce calibration time.

According to one feature of the invention, the reflector is attached on the robotic arm.

Thus, one advantage afforded is that the calibration of the surgical tool can easily be implemented by reducing the distance between the reflector and the robotic arm, compared to a situation where the reflector would be located far away from the robotic arm. In other words, one advantage afforded is that of avoiding to move the robotic arm towards the reflector in order to reduce calibration time.

According to one feature of the invention, the surgical navigation system comprises a cart on which the robotic arm is arranged; wherein the reflector is attached, particularly irreversibly fixed, on the cart.

Thus, one advantage afforded is that of avoiding to move the robotic arm towards the reflector in order to reduce calibration time.

According to one feature of the invention, the reflector comprises a panel having a surface designed to reflect infrared radiations emitted by the light source.

Thus, one advantage afforded is that the panel can easily be implemented with a large surface area.

According to one feature of the invention, the surface of the panel is curved.

Thus, one advantage afforded is that of modulating the distance between the panel and the surgical tool in order to have the same distance between an axis of the surgical tool and the panel.

According to one feature of the invention, the light source is integrated in the camera.

Thus, one advantage afforded is that of improving the compactness of the optical tracking system.

According to one feature of the invention:
- the reflector has a reflective surface defining a half-space;
- the optical tracker is arranged outside the half-space.

Thus, one advantage afforded is that the reflector can be distinguished from the optical tracker in the captured images of the camera.

According to one feature of the invention:
- the reflector has a reflective surface defining a half-space;
- the optical tracker is arranged inside the half-space;
- the optical tracker has markers designed to distinguish the reflector from the optical tracker in the captured images of the camera.

Thus, one advantage afforded is that the optical tracker can be arranged inside the half-space for compactness reasons.

### Definitions

- The term "profile" is understood to mean the outline of the surgical tool observed by one side thereof.
- The term "data processor" is understood to mean a device, such as a calculator or a computer, that performs operations on data.
- The term "pose" is understood to mean the position and the spatial orientation of the surgical tool.
- The term "defined positions" is understood to mean continuous or discontinuous (discrete) positions which are designed to identify the symmetry or the asymmetry of the surgical tool.
- The term "half-space" is understood to mean either of the two parts into which a plane divides Euclidean space. The term "half-space" hereby refers to the part facing the reflecting surface (defining or forming a plane) of the reflector.

### Brief description of the drawings

Other features and advantages will become apparent in the detailed description of various embodiments of the invention, the description being accompanied by examples and references to the appended drawings.
Figure 1 is a partial schematic perspective view of a surgical navigation system according to the invention (the surgical tool is not illustrated) in a surgical room, illustrating the presence of a robotic arm. The surgical room is equipped with an imaging system.
Figure 2 is a schematic perspective view of the robotic arm illustrated in figure 1 on a larger scale.
Figure 3 is a schematic perspective view of the surgical navigation system illustrated in figure 1, with an asymmetrical surgical tool.
Figure 4 is a partial schematic front view of a surgical navigation system according to the invention, illustrating an embodiment where the surgical tool is symmetrical and the holder comprises a carrier designed to carry a universal adapter.
Figure 5 is a schematic view of a captured image of the profile of the surgical tool illustrated in figure 4.
Figure 6 is a partial schematic front view of a surgical navigation system according to the invention, illustrating an embodiment where the surgical tool is asymmetrical and the holder comprises a carrier designed to carry a universal adapter. The surgical tool is mobile with respect to the holder in a set of defined positions which are defined to identify the asymmetry of the surgical tool.
Figure 7 is a partial schematic front view of a surgical navigation system according to the invention, illustrating an embodiment where the surgical tool is asymmetrical and the holder comprises a carrier designed to carry a universal adapter. The holder is mobile to hold the surgical tool in a set of defined positions which are defined to identify the asymmetry of the surgical tool.
Figure 8 is a schematic view of a captured image of the profile of the surgical tool illustrated in figure 6 or figure 7.
Figure 9 is a partial schematic front view of a surgical navigation system according to the invention, illustrating an embodiment where the surgical tool is a Cobb spinal elevator (asymmetrical) and the holder comprises a carrier designed to carry a universal adapter. The Cobb spinal elevator is mobile with respect to the holder in a set of defined positions which are defined to identify the asymmetry of the Cobb spinal elevator.
Figure 10 is a schematic view of a captured image of the profile of the Cobb spinal elevator illustrated in figure 9.
Figure 11 is a view similar to figure 9 illustrating another profile of the Cobb spinal elevator.
Figure 12 is a schematic view of a captured image of the profile of the Cobb spinal elevator illustrated in figure 11.

It should be noted that, for the sake of readability and ease of understanding, the drawings described above are schematic and are not necessarily to scale.

### Detailed description of embodiments

Elements that are identical or provide the same function will carry the same references for the various embodiments, for the sake of simplicity.

One subject of the invention is a surgical navigation system, comprising:
- a surgical tool 1, having a profile 10;
- an optical tracking system, comprising:
   a light source S, configured to emit infrared radiations;
   an optical tracker 2, arranged to track a pose of the surgical tool 1 from a known longitudinal axis A;
   a camera 3, preferably stereoscopic, configured to detect infrared radiations, and arranged to capture images of the optical tracker 2;
   a data processor 4, configured to compute the pose of the surgical tool 1;
- a calibrating device, configured to calibrate the surgical tool 1 tracked by the optical tracking system; the calibrating device comprising:
   a reflector 5, arranged to reflect infrared radiations emitted by the light source S;
   a holder 6, arranged to hold the surgical tool 1 between the light source S and the reflector 5, the holder 6 having a known relative position with respect to the reflector 5;
wherein the light source S is arranged to irradiate the profile 10 of the surgical tool 1 held by the holder 6, the camera 3 is arranged to detect the infrared radiations which are reflected by the surgical tool 1 and by the reflector 5 so as to capture images of the profile 10 of the surgical tool 1, and the data processor 4 is configured to compute the pose of the surgical tool 1 from the captured images of both the optical tracker 2 and the profile 10 of the surgical tool 1.

### Surgical tool

The surgical tool 1 may be symmetrical or asymmetrical. The surgical tool 1 may be an instrument or an implant.

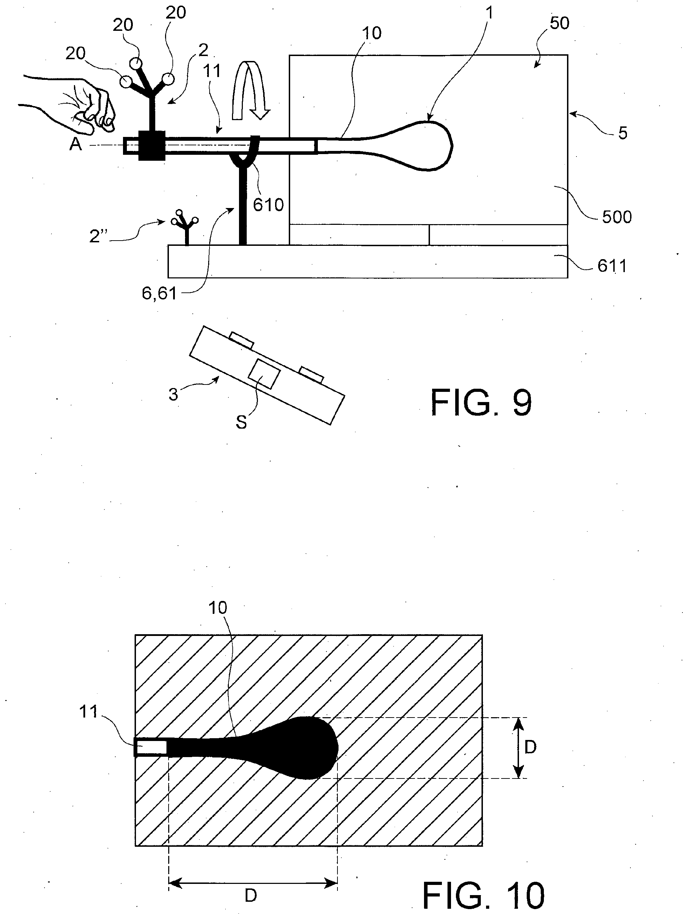

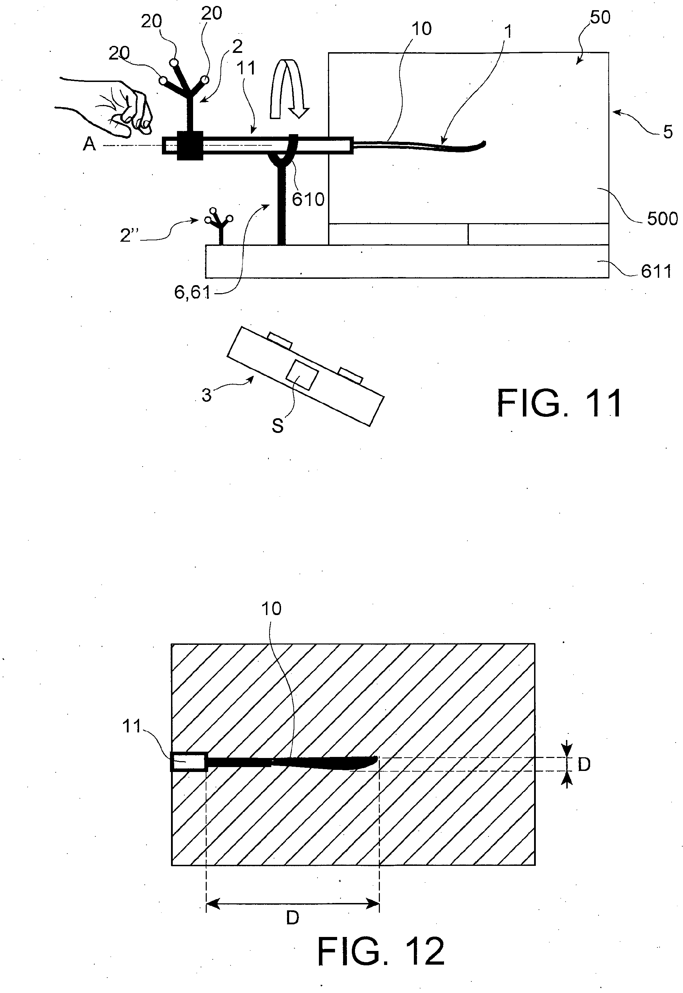

By way of non-limiting example, as illustrated in figures 9 and 11, the surgical tool 1 may be a Cobb spinal elevator which is an asymmetrical tool. A Cobb spinal elevator is a specialized instrument for use in neurosurgical procedures. It can be used in elevation of spinal components in surgeries that require exploration of the vertebral column. For example, it can be used to detach the ligamentum flavum from the lamina or a vertebral disk from the associated vertebrae. Additionally, this elevator features a semi-sharp blade as well as a slight curvature making it well suited for trauma less exploration of delicate soft tissue areas.

As another example, the surgical tool 1 may be a trocar such as a spine trocar. The surgical tool 1 may be a powered surgical tool 1 such as a powered drill, a powered saw, a powered burr or mill, an ultrasonic milling device, a radiofrequency or microwave or cryogenics ablation needle, or any device that can interact with an anatomical structure to be treated.

As other examples, the surgical tool 1 may be an orthopedic implant such as a pedicular screw, a hip implant, a knee implant or a shoulder implant.

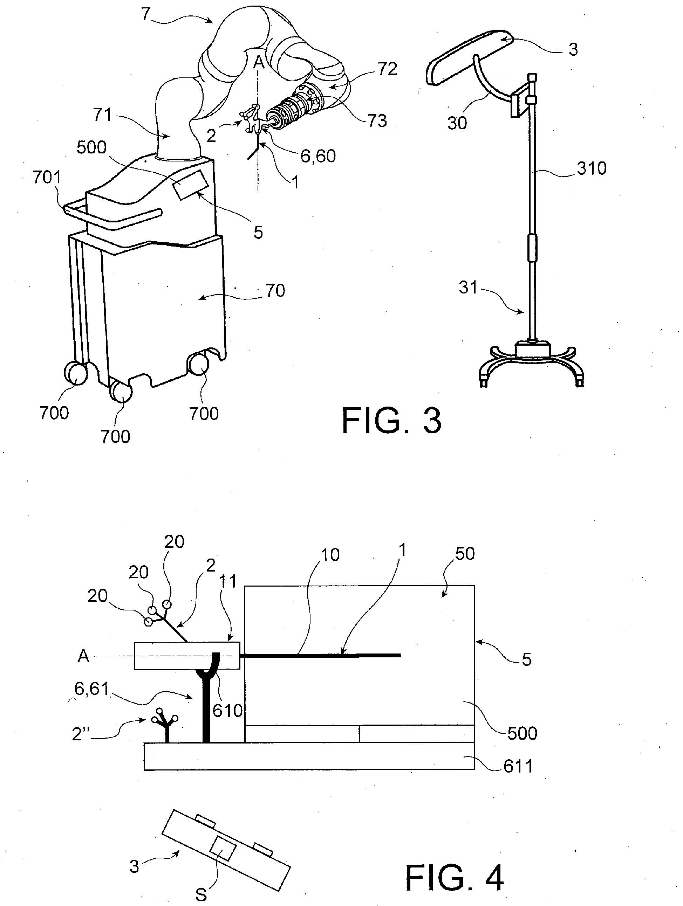

As illustrated in figures 4, 6, 7, 9 and 11, the surgical navigation system may comprise a universal adapter 11 on which the surgical tool 1 is removably mounted. The universal adapter 11 has a known longitudinal axis A which is the axis of symmetry A of the universal adapter 11.

### Robotic arm

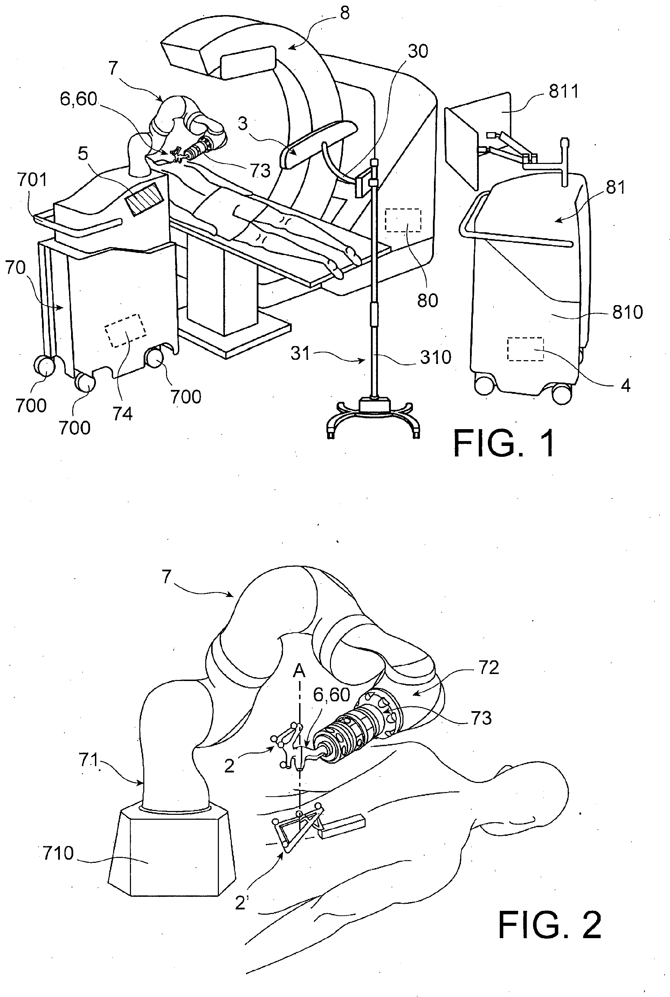

As illustrated in figures 1 to 3, the surgical navigation system may comprise a robotic arm 7 for assisting a user (e.g. a surgeon) during a surgical intervention. For example, in spine surgery, the user may have to implant one or several screws into at least one vertebra. As illustrated in figure 2, a robotic arm 7 may assist the user by holding a drill guide and maintaining the drill guide according to a planned axis. The user may thus use a handheld drill passing through the drill guide held by the robotic arm 7 to drill a hole intended to receive the screw in a vertebra along the planned axis. The patient is equipped with a tracker 2'. The robotic arm 7 may be servo-controlled on the movements of the tracker 2' of the patient in order to maintain alignment with an entry point position on a bone of the patient, compensating for any motion of the bone due to patient's breathing or any mechanical interaction.

The surgical navigation system may comprise a cart 70 on which the robotic arm 7 is arranged. The cart 70 may be mobile on wheels 700, and may include at least one handle 701 to allow an operator to easily maneuver and transport the robotic arm 7. The cart 70 may be manually actuated or, alternatively, motorized with at least one degree of freedom. At least one of the wheels 700 may be blocked once the cart 70 has been moved to the desired position with respect to the operating table. The cart 70 may comprise switches, such as power switches, an emergency button or the like.

The robotic arm 7 has:
- a proximal end 71, extending from a base 710;
- a distal end 72, opposite to the proximal end 71.

The robotic arm 7 may be equipped with an end-effector 73 which is attached at the distal end 72. The end-effector 73 may comprise a hand grip and a tool guide 60. The hand grip is configured to be held by a user's hand for manipulating the robotic arm 7. The hand grip may comprise first and second switches configured to be separately actuated by the user to trigger the operating modes of the robotic arm 7. Said switches may include push-buttons, resistive switches, piezoelectric switches, etc. Although said switches may be arranged in another part of the robotic arm 7, said switches are preferably arranged on the hand grip so that the user may use a single hand to actuate one of said switches and at the same time handle the end-effector 73 either to move the robotic arm 7 (in a hand guiding mode) or to follow the movement of the robotic arm 7 (in a computed trajectory mode). The hand grip may comprise at least one user interface configured to provide information about the current mode. Although said user interface may be arranged in another part of the robotic arm 7, said user interface is advantageously located on the hand grip or on the vicinity thereof so as to allow the user to concentrate on the end-effector 73 when the robotic arm 7 is operated. The user interface may include a display with a changing graphical item (e.g. text, marks with variable color, etc.) depending on the current mode. The user interface may include a plurality of light-emitting diodes configured to have predetermined colors and/or blinking conditions depending on the current mode. Said light-emitting diodes may be arranged as a ring around the hand grip or in the vicinity thereof.

The robotic arm 7 may be equipped with a controller 74, configured to controllably move the robotic arm 7 according to a planned trajectory. The controller 74 may comprise a processor, a data storage device and a communication device. The controller 74 may be configured to controllably move at least part of the cart 70 (e.g. at least one wheel 700). The controller 74 may be embedded in the cart 70. As an alternative, the controller 74 may be provided separate from the cart 70, and may be configured to communicate wirelessly or by wires with the robotic arm 7.

The robotic arm 7 comprises a plurality of degrees of freedom in translation and/or rotation. Usually, the robotic arm 7 comprises at least five, and preferably six or seven motorized degrees of freedom. To that end, the robotic arm 7 comprises a plurality of articulated segments driven by motors. The robotic arm 7 may be for example the LBR Med^{™} robot provided by KUKA (Germany). This robotic arm 7 may be controlled in an autonomous mode according to desired targets and trajectories. As an alternative, this robotic arm 7 may be manipulated using a collaborative mode (cobot). As another alternative, this robotic arm 7 may be telemanipulated using a master control device. A combination of these different modes may be used on this robotic arm 7.

The robotic arm 7 may be active in the sense that it holds and moves a powered surgical tool 1 that interacts directly with an anatomical structure. On the contrary, the robotic arm 7 may be passive in the sense that it holds a tool guide 60 in a predefined position with respect to the anatomical structure into which a powered surgical tool 1 is inserted by a surgeon. For example, a powered drill may be mounted on the tool guide 60 in order to actively drill a bone along a predefined path until an end-point of a selected linear trajectory is reached. As another example, a powered burr can be used to remove a volume of bone where a tumor has been detected, the robotic 7 being controlled to have the burr tip execute a 3D complex path trajectory corresponding to the bone volume to be removed.

### Calibrating device

The calibrating device is configured to calibrate the surgical tool 1 tracked by the optical tracking system. The surgical tool 1 may be pre-operatively or intra-operatively calibrated by the calibrating device.

### Reflector

The reflector 5 is arranged to reflect infrared radiations emitted by the light source S.

The reflector 5 may comprise a reflective surface 500 designed to reflect infrared radiations emitted by the light source S. The reflective surface 500 of the reflector 5 may be flat or curved. The reflective surface 500 of the reflector may be mirror-polished.

Specifically, as illustrated in figures 4, 6, 7, 9 and 11, the reflector 5 may comprise a panel 50 having a reflective surface 500 designed to reflect infrared radiations emitted by the light source S. The reflective surface 500 of the panel 50 may be flat or curved.

The reflector 5 may be attached on the robotic arm 7.

The reflector 5 may be attached, particularly irreversibly fixed, on the cart 70.

The reflector 5 has a reflective surface 500 defining a half-space.

The pose of the reflector 5 may be tracked, preferably by an additional optical tracker 2". The additional optical tracker 2" may be arranged on the robotic arm 7 when the reflector 5 is attached on the cart 70.

### Holder

The holder 6 is arranged to hold the surgical tool 1 between the light source S and the reflector 5. The holder 6 has a known relative position with respect to the reflector 5. For this purpose, the holder 6 may be equipped with a tracker arranged to track the position of the holder 6 with respect to the reflector 5. As an alternative, the reflector 5 may be equipped with fiducials designed to locate the position of the reflector 5 with respect to the holder 6. By way of non-limiting examples, fiducials may be patterns, reliefs, holes, grids, Moiré fringes etc. As previously mentioned, the additional optical tracker 2" can be used to locate the position of the reflector 5 with respect to the holder 6. As an alternative, the holder 6 may be attached on the camera 3. Thus, one advantage afforded is that the position of the holder 6 need not to be tracked because the original position of the camera 3 is known.

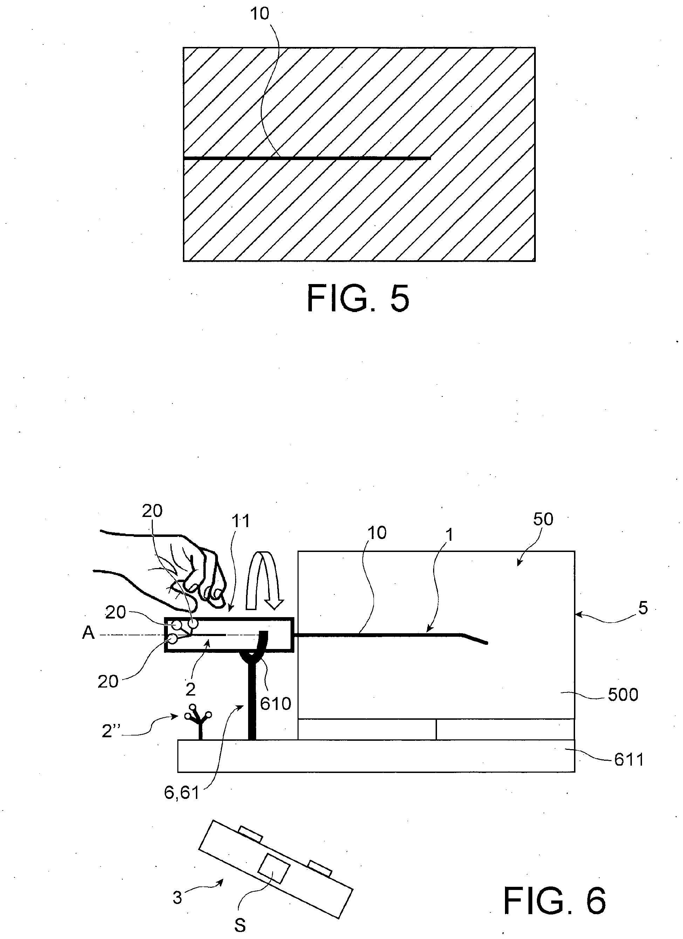

In a first embodiment, as illustrated in figure 6, the surgical tool 1 is mobile with respect to the holder 6 in a set of defined positions which are defined to identify the symmetry or the asymmetry of the surgical tool 1. Specifically, the surgical tool 1 may be mobile in rotation with respect to the holder 6 about the axis of symmetry (the known longitudinal axis A) of the universal adapter 11, when the surgical tool 1 is mounted on the universal adapter 11. Moreover, the surgical tool 1 may be mobile in translation with respect to the holder 6 along the axis of symmetry of the universal adapter 11. Thus, one advantage afforded is that of providing first and second profiles 10 of the surgical tool 1. The first profile 10 is the closest to reality whereas the second profile 10 takes into account a safety margin on the length of surgical tool 1.

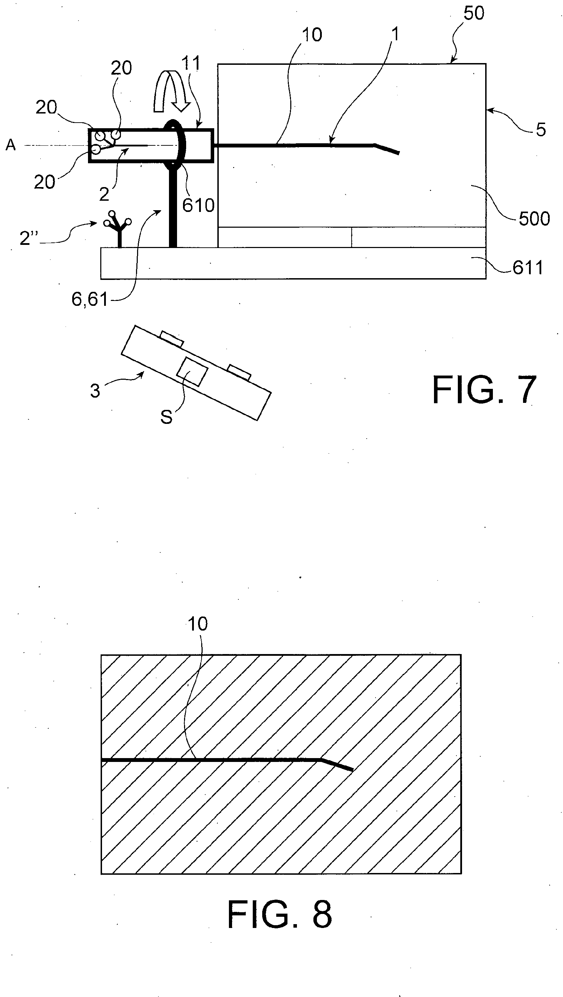

In a second embodiment, as illustrated in figure 7, the holder 6 is mobile to hold the surgical tool 1 in a set of defined positions which are defined to identify the symmetry or the asymmetry of the surgical tool 1. The holder 6 is preferably mobile in rotation about three axes defining a rectangular trihedron. Specifically, the holder 6 may be mobile in rotation with respect to the surgical tool 1 about the axis of symmetry (the known longitudinal axis A) of the universal adapter 11, when the surgical tool 1 is mounted on the universal adapter 11.

As illustrated primarily in figures 2 and 3, the holder 6 may comprise a tool guide 60 mounted on the robotic arm 7. Specifically, the tool guide 60 may be mounted on the end-effector 73 of the robotic arm 7. The tool guide 60 extends along a guiding axis A so as to guide the surgical tool 1. The surgical tool 1 may be movable relative to the tool guide 60 in rotation and/or translation about said guiding axis A. In this case, the tool guide 60 comprises a tool channel into which the surgeon can insert the surgical tool 1 in order to proceed to the surgery. The tool guide 60 may be placed with a given position and orientation such that when the surgeon inserts the surgical tool 1 into the tool guide 60, the position and orientation of the surgical tool 1 coincides with a defined surgical target. The tool guide 60 orients the surgical tool 1 along the tool guide axis A so as to guide a translation of a surgical tool 1 along the tool guide axis A. The position of the surgical tool 1 along the tool guide axis A is controlled by the surgeon. The tool guide 60 may also contain a stop adapted to limit the depth of the surgical tool 1. Therefore, the user cannot insert the surgical tool 1 deeper than a predetermined depth. Alternatively, the surgical tool 1 may be fixed to the tool guide 60; for example, the tool guide 60 may comprise a tool holder comprising a support and fastening means configured to rigidly fix the surgical tool 1 to the support. Optionally, the tool guide 60 may also comprise one or more actuators adapted to move the tool holder and the surgical tool in translation and/or in rotation about the guiding axis A relative to the robotic arm 7. The tool guide 60 may be placed with a given position and orientation such that the position and orientation of the surgical tool 1 may coincide with a surgical target. Both the position and the orientation of the surgical tool 1 along the tool guide axis A are controlled by the robotic arm 7 and/or the actuator(s). The tool guide 60 may be removably or permanently attached to the robotic arm 7. A passive tool guide 60 is configured to hold a guide in which slides a surgical tool 1, which may be a drill guide, a drill, a cutting guide, or any active tool. An active tool guide 60 is configured to hold an active surgical tool 1, which may be a drill, an ultrasonic cutter, a burr, or any active tool. The active tool guide 60 may also hold an actuator comprising several motorized degrees of freedom so as to move and activate an active surgical tool 1.

A surgical target may be defined by a surgical target axis. The surgical target axis may correspond to a given surgical target axis position and/or orientation. Aligning the tool guide 60 with the surgical target axis corresponds to placing the tool guide 60 on the surgical target axis. In other words, when aligned, the position and/or orientation of the tool guide 60 correspond to the position and/or orientation of the surgical target axis. More particularly, the surgical target axis may correspond to a desired position and/or orientation of the tool guide axis A and of the surgical tool 1 axis when the surgical tool 1 is inserted in the tool channel, or fixed to the tool guide 60. For example, in the case where the surgical tool 1 is a drill, the surgical target may correspond to the axis along which the hole is drilled.

As illustrated in figures 4, 6, 7, 9 and 11, the holder 6 may comprise a carrier 61 designed to carry the universal adapter 11. The carrier 61 may include a ring-shaped end portion 610 arranged to receive the universal adapter 11. The ring-shaped end portion 610 of the carrier may partially or totally surround the universal adapter 11. Specifically, the carrier 61 may be arranged on a base 611 to provide stability by the weight thereof. As previously mentioned, the pose of the reflector 5 may be tracked, preferably by an additional optical tracker 2". The additional optical tracker 2" may be attached on the base 611. As an alternative, the carrier 61 may be mounted on the robotic arm 7.

As an alternative, the universal adapter 11 may be mounted on the tool guide 60.

### Optical tracking system

### Light source & Camera

The light source S is configured to emit infrared radiations. The light source S may comprise a set of light-emitting diodes. The light source S is advantageously integrated in the camera 3 for compactness reasons. As an alternative, the light source S may belong to a dedicated infrared illuminator.

The camera 3 is configured to detect infrared radiations, and arranged to capture images of the optical tracker 2. The camera 3 is arranged to detect the infrared radiations which are reflected by the surgical tool 1 and by the reflector 5 so as to capture images of the profile 10 of the surgical tool 1. The captured images of the profile 10 of the surgical tool 1 may be 2D images in shades of gray or in black and white (by applying a saturation threshold). The profile 10 of the surgical tool 1 can be observed in the captured images of the camera 3 insofar as the reflection coefficient of the reflector 5 (dedicated to this function) is much higher than the reflection coefficient of the surgical tool 1. Thus, the infrared rays emitted by the light source S and reflected by the surgical tool 1 result in a black pixel on captured images. On the contrary, the infrared rays emitted by the light source S and reflected by the reflector 5 result in a white pixel on captured images. Schematic examples of captured images are illustrated in figures 5, 8, 10 and 12.

The camera 3 is advantageously stereoscopic for compactness reasons and to provide depth information. As an alternative, the surgical navigation system may comprise two cameras 3 configured to detect infrared radiations, and arranged to capture images of the optical tracker so as to provide depth information. As illustrated in figures 1 and 3, the camera 3 may comprise a support 30 mounted on a rolling stand 31. The rolling stand 31 may include a telescopic rod 310 defining an axis. The support 30 of the camera 3 is advantageously mobile in rotation about the axis of the telescopic rod 310. The support 30 of the camera 3 is advantageously mobile in translation along the axis of the telescopic rod 310.

### Optical tracker

The optical tracker 2 is arranged to track a pose of the surgical tool 1 from a known longitudinal axis A. The optical tracker 2 may comprise a set of reflective markers 20. The set of reflective markers 20 may have a spherical shape. The reflective markers 20 are designed to reflect infrared radiations. The pose of the surgical tool 1 can be defined by three parameters of position and three parameters of orientation.

If the surgical tool 1 has a known axis of symmetry A, the optical tracker 2 may be arranged on the surgical tool 1 to track a pose of the surgical tool 1 from a known longitudinal axis A which is the axis of symmetry A of the surgical tool 1. The optical tracker 2 may be removably or permanently attached on the surgical tool 1, for example by a mechanical or a magnetic attachment.

The optical tracker 2 may be arranged on the tool guide 60 to track a pose of the surgical tool 1 from a known longitudinal axis A which is the guiding axis A of the tool guide 60. The optical tracker 2 may be removably or permanently attached on the tool guide 60, for example by a mechanical or a magnetic attachment. In this embodiment, the surgical navigation system may comprise a universal adapter 11 on which the surgical tool 1 is removably mounted. The universal adapter 11 may be mounted on the tool guide 60. The universal adapter 11 may lack an optical tracker 2. In this case, the optical tracker 2 is arranged on the tool guide 60 to track a pose of the surgical tool 1 from a known longitudinal axis A which is the guiding axis A of the tool guide 60.

The optical tracker 2 may extend over the universal adapter 11 to track a pose of the surgical tool 1 from a known longitudinal axis A which is the axis of symmetry A of the universal adapter 11. The optical tracker 2 is preferably permanently attached on the universal adapter 11. The optical tracker 2 and the universal adapter 11 are preferably single-piece. When the universal adapter 11 is mounted on the tool guide 60, the optical tracker 2 may be arranged on the universal adapter 11 to track a pose of the surgical tool 1 from a known longitudinal axis A which is preferably the axis of symmetry A of the universal adapter 11 instead of the guiding axis A of the tool guide 60. The axis of symmetry A of the universal adapter 11 may coincide with the guiding axis A of the tool guide 60.

In a first embodiment, the optical tracker 2 is arranged outside the half-space defined by the reflective surface 500 of the reflector 5.

In a second embodiment, the optical tracker 2 is arranged inside said half-space and the optical tracker 2 has markers 20 designed to distinguish the reflector 5 from the optical tracker 2 in the captured images of the camera 3. For this purpose, markers 20 may include a non-reflecting surface (e.g. by obscuring) or holes.

### Data processor

The data processor 4 is configured to compute the pose of the surgical tool 1 from the captured images of both the optical tracker 2 and the profile 10 of the surgical tool 1.

Specifically, the data processor 4 may be configured to process the 2D captured images of the camera 3 to segment out the profile 10 of the surgical too1 1. The pose of the surgical tool 1 can be computed by using a matrix transformation between first and second coordinate systems. The first coordinate system is fixed with respect to the profile 10 of the surgical tool 1, whereas the second coordinate system is fixed with respect to the optical tracker 2. The matrix transformation is a rigid transformation referring to geometric transformation of a Euclidean space that preserves the Euclidean distance between every pair of points. The rigid transformation may include rotations, translations, reflections or a combination thereof. As illustrated in figures 10 and 12, the data processor 4 may be configured to extract characteristic dimensions D of the profile 10 of the surgical tool 1 from the captured images of both the optical tracker 2 and the profile 10 of the surgical tool 1.

If the surgical tool 1 is mobile with respect to the holder 6 in a set of defined positions which are defined to identify the symmetry or the asymmetry of the surgical tool 1, then the data processor 4 is advantageously configured to compute the pose of the surgical tool 1 from the captured images of both the optical tracker 2 and a set of profiles 10 of the surgical tool 1, the set of profiles 10 corresponding to the set of defined positions.

If the holder 6 is mobile to hold the surgical tool 1 in a set of defined positions which are defined to identify the symmetry or the asymmetry of the surgical tool 1, then the data processor 4 is advantageously configured to compute the pose of the surgical tool 1 from the captured images of both the optical tracker 2 and a set of profiles 10 of the surgical tool 1, the set of profiles 10 corresponding to the set of defined positions.

For a symmetrical surgical tool 1, the first image captured by the camera 3 give enough information to identify the geometrical profile 10 of the surgical tool. Moving the surgical tool 1 (or the holder 6) in another defined position (e.g. by rotation) would not provide more information about the profile 10 of the surgical tool 1; however it would detect whether the surgical tool 1 extends along the known axis of symmetry of the universal adapter 11, or whether the geometrical profile 10 follows a theoretical profile.

For an asymmetrical surgical tool 1, at least a second image has to be captured by the camera 3 after moving the surgical tool 1 (or the holder 6) in another defined position (e.g. by rotation). The more captured images, the better the accuracy of the profile 10 of the surgical tool 1.

### Imaging system

As illustrated in figure 1, the surgical navigation system may comprise an X-ray imaging system. X-Ray imaging systems are frequently used during surgical procedures to provide physicians with image-based information about a patient's anatomical situation and/or the position and orientation of the surgical tool 1 with respect to the patient.

Such X-ray imaging systems typically provide two-dimensional (2D) projection images with different anatomical structures superimposed along the path of the X-rays.

A typical example of such a system for use in an intra-operative setting is the so-called C-arm 8 which comprises a base frame on which a C-shaped arm 8 is attached with several intermediate joints allowing moving the C-shaped arm 8 in space along several degrees of freedom. One end of the C-shaped arm 8 carries an X-ray source and the other end carries an image detector. In other words, the X-ray source and the X-ray image detector are carried by a C-shaped gantry, the X-ray source and the X-ray image detector being arranged on opposite ends of the gantry. Due to the shape of the gantry, such an imaging system is usually called a C-arm.

The reflector 5 may be attached on the C-shaped arm 8.

Due to the limited information provided by these 2D images, three-dimensional (3D) imaging techniques have become necessary over the past decades.

While computer tomography is a well-established class of stationary X-ray imaging systems used for 3D reconstruction in a radiology department, these devices are in general not usable inside the operating room.

Recent years have seen an increasing interest in tomographic reconstruction techniques also known as cone-beam reconstruction techniques (CBCT), using two-dimensional detectors.

Special efforts have been made to enable the abovementioned C-shaped arms 8 to provide three-dimensional information by automatically acquiring a set of 2D images and subsequently reconstructing a 3D image based on said cone-beam reconstruction techniques.

In a manner known per se, the X-ray imaging system may be configured to produce at least one 2D X-ray image that is the result of a conical projection of a patient anatomy, wherein the tip of the cone is approximately the central point of the X-ray source and the basis of the cone is approximately the portion of the X-ray image detector that is reached by X-ray beams that have been collimated in a given shape and orientation.

The X-ray imaging system may be equipped with a controller 80 configured to controllably move the C-shaped arm 8 according to a planned trajectory. The controller 80 may comprise a processor, a data storage device and a communication device. The controller 80 is advantageously embedded in a base of the C-shaped arm 8.

The X-ray imaging system may comprise a surgical station 81. The surgical station 81 may include a mobile trolley 810 that supports:
- at least one user monitor 811,
- at least one user interface (not shown),
- the data processor 4, which may also be configured to process captured images of the X-ray image detector.

The reflector 5 may be attached on the surgical station 81.

## Claims

1. A surgical navigation system, comprising:
- a surgical tool (1), having a profile (10);
- an optical tracking system, comprising:
a light source (S), configured to emit infrared radiations;
an optical tracker (2), arranged to track a pose of the surgical tool (1) from a known longitudinal axis (A);
a camera (3), preferably stereoscopic, configured to detect infrared radiations, and arranged to capture images of the optical tracker (2);
a data processor (4), configured to compute the pose of the surgical tool (1);
- a calibrating device, configured to calibrate the surgical tool (1) tracked by the optical tracking system; the calibrating device comprising:
a reflector (5), arranged to reflect infrared radiations emitted by the light source (S);
a holder (6), arranged to hold the surgical tool (1) ;
**characterised in that** the holder (6) is arranged to hold the surgical tool (1) between the light source (S) and the reflector (5), the holder (6) having a known relative position with respect to the reflector (5); wherein the light source (S) is arranged to irradiate the profile of the surgical tool (1) held by the holder (6), the camera (3) is arranged to detect the infrared radiations which are reflected by the surgical tool (1) and by the reflector (5) so as to capture images of the profile (10) of the surgical tool (1), and the data processor (4) is configured to compute the pose of the surgical tool (1) from the captured images of both the optical tracker (2) and the profile (10) of the surgical tool (1).

2. The surgical navigation system according to claim 1, wherein:
- the surgical tool (1) is mobile with respect to the holder (6) in a set of defined positions which are defined to identify a symmetry or an asymmetry of the surgical tool (1);
- the data processor (4) is configured to compute the pose of the surgical tool (1) from the captured images of both the optical tracker (2) and a set of profiles (10) of the surgical tool (1), the set of profiles (10) corresponding to the set of defined positions.

3. The surgical navigation system according to claim 1, wherein:
- the holder (6) is mobile to hold the surgical tool (1) in a set of defined positions which are defined to identify the symmetry or the asymmetry of the surgical tool (1), the holder (6) is preferably mobile in rotation about three axes defining a rectangular trihedron;
- the data processor (4) is configured to compute the pose of the surgical tool (1) from the captured images of both the optical tracker (2) and a set of profiles (10) of the surgical tool (1), the set of profiles (10) corresponding to the set of defined positions.

4. The surgical navigation system according to any claim 1 to 3, wherein:
- the surgical tool (1) has a known axis of symmetry;
- the known longitudinal axis (A) is the axis of symmetry of the surgical tool (1);
- the optical tracker (2) is arranged on the surgical tool (1).

5. The surgical navigation system according to any claim 1 to 3, comprising a robotic arm (7); wherein:
- the holder (6) comprises a tool guide (60) mounted on the robotic arm (7), the tool guide (60) extending along a guiding axis so as to guide the surgical tool (1);
- the known longitudinal axis (A) is the guiding axis of the tool guide (60);
- the optical tracker (2) is arranged on the tool guide (60).

6. The surgical navigation system according to any claim 1 to 3, comprising a universal adapter (11) on which the surgical tool (1) is removably mounted; wherein:
- the universal adapter (11) has a known axis of symmetry;
- the known longitudinal axis (A) is the axis of symmetry of the universal adapter (11);
- the optical tracker (2) extends over the universal adapter (11).

7. The surgical navigation system according to claim 6, wherein the holder (6) comprises a carrier (61) designed to carry the universal adapter (11).

8. The surgical navigation system according to claim 7, comprising a robotic arm (7); wherein the carrier (61) is mounted on the robotic arm (7).

9. The surgical navigation system according to claim 5 or 8, wherein the reflector (5) is attached on the robotic arm (7).

10. The surgical navigation system according to claim 5 or 8, comprising a cart (70) on which the robotic arm (7) is arranged; wherein the reflector (5) is attached, particularly irreversibly fixed, on the cart (70).

11. The surgical navigation system according to any claim 1 to 10, wherein the reflector (5) comprises a panel (50) having a reflective surface (500) designed to reflect infrared radiations emitted by the light source (S).

12. The surgical navigation system according to claim 11, wherein the reflective surface (500) of the panel (50) is curved.

13. The surgical navigation system according to any claim 1 to 12, wherein the light source (S) is integrated in the camera (3).

14. The surgical navigation system according to any claim 1 to 13, wherein:
- the reflector (5) has a reflective surface (500) defining a half-space;
- the optical tracker (2) is arranged outside the half-space.

15. The surgical navigation system according to any claim 1 to 13, wherein:
- the reflector (5) has a reflective surface (500) defining a half-space;
- the optical tracker (2) is arranged inside the half-space;
- the optical tracker (2) has markers (20) designed to distinguish the reflector (5) from the optical tracker (2) in the captured images of the camera (3).

## Patentansprüche

1. Chirurgisches Navigationssystem, umfassend:
- ein chirurgisches Werkzeug (1), das ein Profil (10) aufweist;
- ein optisches Verfolgungssystem, umfassend:
eine Lichtquelle (S), die dazu ausgelegt ist, Infrarotstrahlungen zu emittieren;
einen optischen Tracker (2), angeordnet, um eine Pose des chirurgischen Werkzeugs (1) anhand einer bekannten Längsachse (A) zu verfolgen;
eine Kamera (3), vorzugsweise stereoskopisch, die dazu ausgelegt ist, Infrarotstrahlungen zu detektieren, und angeordnet ist, um Bilder des optischen Trackers (2) zu erfassen;
einen Datenprozessor (4), der dazu ausgelegt ist, die Pose des chirurgischen Werkzeugs (1) zu berechnen;
- eine Kalibriervorrichtung, die dazu ausgelegt ist, das von dem optischen Verfolgungssystem verfolgte chirurgische Werkzeug (1) zu kalibrieren; wobei die Kalibriervorrichtung umfasst:
einen Reflektor (5), angeordnet, um von der Lichtquelle (S) emittierte Infrarotstrahlungen zu reflektieren;
eine Halterung (6), angeordnet, um das chirurgische Werkzeug (1) zu halten;
**dadurch gekennzeichnet, dass** die Halterung (6) angeordnet ist, um das chirurgische Werkzeug (1) zwischen der Lichtquelle (S) und dem Reflektor (5) zu halten, wobei die Halterung (6) eine bekannte relative Position im Hinblick auf den Reflektor (5) hat; wobei die Lichtquelle (S) angeordnet ist, um das Profil des von der Halterung (6) gehaltenen chirurgischen Werkzeugs (1) zu bestrahlen, die Kamera (3) angeordnet ist, um die Infrarotstrahlungen zu detektieren, die von dem chirurgischen Werkzeug (1) und von dem Reflektor (5) reflektiert werden, sodass Bilder des Profils (10) des chirurgischen Werkzeugs (1) erfasst werden, und der Datenprozessor (4) dazu ausgelegt ist, die Pose des chirurgischen Werkzeugs (1) anhand der erfassten Bilder des optischen Trackers (2) und des Profils (10) des chirurgischen Werkzeugs (1) zu berechnen.

2. Chirurgisches Navigationssystem gemäß Anspruch 1, wobei:
- das chirurgische Werkzeug (1) im Hinblick auf die Halterung (6) in einem Satz von definierten Positionen, die definiert sind, um eine Symmetrie oder eine Asymmetrie des chirurgischen Werkzeugs (1) zu identifizieren, beweglich ist;
- der Datenprozessor (4) dazu ausgelegt ist, die Pose des chirurgischen Werkzeugs (1) anhand der erfassten Bilder des optischen Trackers (2) sowie einem Satz von Profilen (10) des chirurgischen Werkzeugs (1) zu berechnen, wobei der Satz von Profilen (10) dem Satz von definierten Positionen entspricht.

3. Chirurgisches Navigationssystem gemäß Anspruch 1, wobei:
- die Halterung (6) beweglich ist, um das chirurgische Werkzeug (1) in einem Satz von definierten Positionen zu halten, die definiert sind, um die Symmetrie oder Asymmetrie des chirurgischen Werkzeugs (1) zu identifizieren, wobei die Halterung (6) vorzugsweise drehbeweglich um drei Achsen ist, die ein rechtwinkliges Trieder definieren;
- der Datenprozessor (4) dazu ausgelegt ist, die Pose des chirurgischen Werkzeugs (1) anhand der erfassten Bilder des optischen Trackers (2) sowie einem Satz von Profilen (10) des chirurgischen Werkzeugs (1) zu berechnen, wobei der Satz von Profilen (10) dem Satz von definierten Positionen entspricht.

4. Chirurgisches Navigationssystem gemäß einem der Ansprüche 1 bis 3, wobei:
- das chirurgische Werkzeug (1) eine bekannte Symmetrieachse aufweist;
- die bekannte Längsachse (A) die Symmetrieachse des chirurgischen Werkzeugs (1) ist;
- der optische Tracker (2) an dem chirurgischen Werkzeug (1) angeordnet ist.

5. Chirurgisches Navigationssystem gemäß einem der Ansprüche 1 bis 3, umfassend einen Roboterarm (7); wobei:
- die Halterung (6) eine Werkzeugführung (60) umfasst, die an dem Roboterarm (7) montiert ist, wobei sich die Werkzeugführung (60) entlang einer Führungsachse erstreckt, sodass das chirurgische Werkzeug (1) geführt wird;
- die bekannte Längsachse (A) die Führungsachse der Werkzeugführung (60) ist;
- der optische Tracker (2) an der Werkzeugführung (60) angeordnet ist.

6. Chirurgisches Navigationssystem gemäß einem der Ansprüche 1 bis 3, umfassend einen Universaladapter (11), an dem das chirurgische Werkzeug (1) abnehmbar montiert ist; wobei:
- der Universaladapter (11) eine bekannte Symmetrieachse aufweist;
- die bekannte Längsachse (A) die Symmetrieachse des Universaladapters (11) ist;
- sich der optische Tracker (2) über den Universaladapter (11) erstreckt.

7. Chirurgisches Navigationssystem gemäß Anspruch 6, wobei die Halterung (6) einen Träger (61) umfasst, der ausgestaltet ist, um den Universaladapter (11) aufzunehmen.

8. Chirurgisches Navigationssystem gemäß Anspruch 7, umfassend einen Roboterarm (7); wobei der Träger (61) an dem Roboterarm (7) montiert ist.

9. Chirurgisches Navigationssystem gemäß Anspruch 5 oder 8, wobei der Reflektor (5) an dem Roboterarm (7) angebracht ist.

10. Chirurgisches Navigationssystem gemäß Anspruch 5 oder 8, umfassend einen Wagen (70), an dem der Roboterarm (7) angeordnet ist; wobei der Reflektor (5) an dem Wagen (70) angebracht, insbesondere irreversibel fixiert ist.

11. Chirurgisches Navigationssystem gemäß einem der Ansprüche 1 bis 10, wobei der Reflektor (5) eine Platte (50) mit einer reflektierenden Oberfläche (500) umfasst, die ausgestaltet ist, um von der Lichtquelle (S) emittierte Infrarotstrahlungen zu reflektieren.

12. Chirurgisches Navigationssystem gemäß Anspruch 11, wobei die reflektierende Oberfläche (500) der Platte (50) gekrümmt ist.

13. Chirurgisches Navigationssystem gemäß einem der Ansprüche 1 bis 12, wobei die Lichtquelle (S) in die Kamera (3) integriert ist.

14. Chirurgisches Navigationssystem gemäß einem der Ansprüche 1 bis 13, wobei:
- der Reflektor (5) eine reflektierende Oberfläche (500) aufweist, die einen Halbraum definiert;
- der optische Tracker (2) außerhalb des Halbraums angeordnet ist.

15. Chirurgisches Navigationssystem gemäß einem der Ansprüche 1 bis 13, wobei:
- der Reflektor (5) eine reflektierende Oberfläche (500) aufweist, die einen Halbraum definiert;
- der optische Tracker (2) innerhalb des Halbraums angeordnet ist;
- der optische Tracker (2) Marker (20) aufweist, die ausgestaltet sind, um den Reflektor (5) von dem optischen Tracker (2) in den erfassten Bildern der Kamera (3) zu unterscheiden.

## Revendications

1. Système de navigation chirurgicale, comprenant :
- un outil chirurgical (1), ayant un profil (10) ;
- un système de suivi optique, comprenant :
une source de lumière (S), configurée pour émettre des radiations infrarouges ;
un dispositif de suivi optique (2), agencé pour suivre une pose de l'outil chirurgical (1) à partir d'un axe longitudinal connu (A) ;
une caméra (3), de préférence stéréoscopique, configurée pour détecter des radiations infrarouges, et agencée pour capturer des images du dispositif de suivi optique (2) ;
un processeur de données (4), configuré pour calculer la pose de l'outil chirurgical (1) ;
- un dispositif d'étalonnage, configuré pour étalonner l'outil chirurgical (1) suivi par le système de suivi optique ; le dispositif d'étalonnage comprenant :
un réflecteur (5), agencé pour réfléchir des radiations infrarouges émises par la source de lumière (S) ;
un support (6), agencé pour maintenir l'outil chirurgical (1) ;
**caractérisé en ce que** le support (6) est agencé pour maintenir l'outil chirurgical (1) entre la source de lumière (S) et le réflecteur (5), le support (6) ayant une position relative connue par rapport au réflecteur (5) ; la source de lumière (S) étant agencée pour irradier le profil de l'outil chirurgical (1) maintenu par le support (6), la caméra (3) étant agencée pour détecter les radiations infrarouges qui sont réfléchies par l'outil chirurgical (1) et par le réflecteur (5) de manière à capturer des images du profil (10) de l'outil chirurgical (1), et le processeur de données (4) étant configuré pour calculer la pose de l'outil chirurgical (1) à partir des images capturées à la fois du dispositif de suivi optique (2) et du profil (10) de l'outil chirurgical (1).

2. Système de navigation chirurgicale selon la revendication 1,
- l'outil chirurgical (1) étant mobile par rapport au support (6) dans un ensemble de positions définies qui sont définies pour identifier une symétrie ou une asymétrie de l'outil chirurgical (1) ;
- le processeur de données (4) étant configuré pour calculer la pose de l'outil chirurgical (1) à partir des images capturées à la fois du dispositif de suivi optique (2) et d'un ensemble de profils (10) de l'outil chirurgical (1), l'ensemble de profils (10) correspondant à l'ensemble de positions définies.

3. Système de navigation chirurgicale selon la revendication 1,
- le support (6) étant mobile pour maintenir l'outil chirurgical (1) dans un ensemble de positions définies qui sont définies pour identifier la symétrie ou l'asymétrie de l'outil chirurgical (1), le support (6) étant de préférence mobile en rotation autour de trois axes définissant un trièdre rectangulaire ;
- le processeur de données (4) étant configuré pour calculer la pose de l'outil chirurgical (1) à partir des images capturées à la fois du dispositif de suivi optique (2) et d'un ensemble de profils (10) de l'outil chirurgical (1), l'ensemble de profils (10) correspondant à l'ensemble de positions définies.

4. Système de navigation chirurgicale selon l'une quelconque des revendications 1 à 3,
- l'outil chirurgical (1) ayant un axe de symétrie connu ;
- l'axe longitudinal connu (A) étant l'axe de symétrie de l'outil chirurgical (1) ;
- le dispositif de suivi optique (2) étant agencé sur l'outil chirurgical (1).

5. Système de navigation chirurgicale selon l'une quelconque des revendications 1 à 3, comprenant un bras robotique (7),
- le support (6) comprenant un guide d'outil (60) monté sur le bras robotique (7), le guide d'outil (60) s'étendant le long d'un axe de guidage de manière à guider l'outil chirurgical (1) ;
- l'axe longitudinal connu (A) étant l'axe de guidage du guide d'outil (60) ;
- le dispositif de suivi optique (2) étant agencé sur le guide d'outil (60).

6. Système de navigation chirurgicale selon l'une quelconque des revendications 1 à 3, comprenant un adaptateur universel (11) sur lequel l'outil chirurgical (1) est monté de manière amovible,
- l'adaptateur universel (11) ayant un axe de symétrie connu ;
- l'axe longitudinal connu (A) étant l'axe de symétrie de l'adaptateur universel (11) ;
- le dispositif de suivi optique (2) s'étendant sur l'adaptateur universel (11).

7. Système de navigation chirurgicale selon la revendication 6, le support (6) comprenant un porteur (61) conçu pour porter l'adaptateur universel (11).

8. Système de navigation chirurgicale selon la revendication 7, comprenant un bras robotique (7) ; le porteur (61) étant monté sur le bras robotique (7).

9. Système de navigation chirurgicale selon la revendication 5 ou 8, le réflecteur (5) étant fixé sur le bras robotique (7).

10. Système de navigation chirurgicale selon la revendication 5 ou 8, comprenant un chariot (70) sur lequel le bras robotique (7) est agencé ; le réflecteur (5) étant fixé, particulièrement fixé de manière irréversible, sur le chariot (70).

11. Système de navigation chirurgicale selon l'une quelconque des revendications 1 à 10, le réflecteur (5) comprenant un panneau (50) ayant une surface réfléchissante (500) conçue pour réfléchir les radiations infrarouges émises par la source de lumière (S).

12. Système de navigation chirurgicale selon la revendication 11, la surface réfléchissante (500) du panneau (50) étant courbée.

13. Système de navigation chirurgicale selon l'une quelconque des revendications 1 à 12, la source de lumière (S) étant intégrée dans la caméra (3).

14. Système de navigation chirurgicale selon l'une quelconque des revendications 1 à 13,
- le réflecteur (5) ayant une surface réfléchissante (500) définissant un demi-espace ;
- le dispositif de suivi optique (2) étant agencé à l'extérieur du demi-espace.

15. Système de navigation chirurgicale selon l'une quelconque des revendications 1 à 13,
- le réflecteur (5) ayant une surface réfléchissante (500) définissant un demi-espace ;
- le dispositif de suivi optique (2) étant agencé à l'intérieur du demi-espace ;
- le dispositif de suivi optique (2) ayant des marqueurs (20) conçus pour distinguer le réflecteur (5) du dispositif de suivi optique (2) dans les images capturées de la caméra (3).
